# EUROPEAN PATENT APPLICATION

(11) **EP 1 293 498 A1**
(43) Date of publication of application: **19.03.2003**
(21) Application number: 01810842.3
(22) Date of filing: 30.08.2001
(51) Int. Cl.: C07C 391/00, A61K 31/095

(54) **Selenium complex with haloethanoic acid or its anhydride, and use thereof in the topical treatment of neoplasms**

(71) Applicant: Mardi, Shalva, 4102 Binningen (CH)
(72) Inventor: Mardi, Shalva., CH-4102 Binningen, Basel land. (CH); Mardi, Rosa., 55400 Kiron. (IL); Mardi, Gymsher., CH-4410 Leistal, Basel-land (CH); Mardi, Laura., CH-4051 Basel. (CH)

(57) **Abstract**

This invention relates to the novel Selenium compounds of aliphatic halocarboxyl acid complex hydrate and anhydrids or their mixture which are useful as an topical agent for the treatment of all types of viral, benign, premalignant and nonmetastasising malignant lesions of the skin and visible mucous membrane, the Selenium complexes are represented by the general formulas N^{o} 1 and N^{o} 2.

CHEMICAL FORMULA*

1. { Se (CXₙH₃ₙCOO)_{y}}⁻zH⁺

2. { Se((CXₙH₃₋ₙCO)₂O)ₖ}

X = F; Cl; Br; I.
n = 0; 1; 2; 3.
y = 4 or 6.
z = 2 or 4.
k = 2 or 3.

## Description

### 1. BACKGROUND OF THE INVENTION

### A) Field of the Invention

This invention relates to novel Selenium complexes only for topical use having antineoplastic activities for the treatment of all types of viral, benign, premalignant and carefully selected nonmetastasising malignant skin and visible mucous membrane lesions .

### B). Description of the prior Art

It has already been reported that a certain type of Selenium compound have an antineoplastic and anticarcinogenic activity /see, for example Japanese Patent: 1. Kokai ( Laid-open ) Nos, 20271 /84 and EP ― 95663 A. 2. USA Patent: Ekimoto, et al. Nº 4,824,955, April 25, 1989; 3. Abstracts of 7-th International Symposium of Selenium in biology and Medicine; Venezia, Italy, October 1 ― 5, 2000./.

After carefully study of scientific and clinical literature as well as Patents, however, no with similar chemical formulation Selenium compound or complexes has yet found which is practically useble for topical treatment all cinical indications, presented in formulas: N 1 and N 2.

### SUMMARY OF THE INVENTION

### Detailed description of the invention

The invention represented by general chemical formulas : Nº1 and Nº 2. In the above-shown general formulas the Halogen ( X.) atom can be Fluorine ( F ), Clorine ( Cl ), Bromine ( Br ), or Iodine ( I ), but Fluorine and Clorine are preferred. The aliphatic carboxyl acids, for instance such as formic, ethanoic, propionic,butaric, valeric, caproic, caprylac, capril, lauric, myristic, palmatic, stearic can be represented, but we prefer ethanoic acid, because molecules are polar and can form hydrogen bonds with each other as well as with other inorganic and organic ingridients to form di-, olygo- or polymer substances.
This invention represented by general formulas 1 and 2 can be obtained from the following reaction between aliphatic carboxyl acids ( hydrate or anhydrids ) and Selenium containing substances, for instance: Selenium metal in powder ( black, gray, red, cristallic ), gas ― SeH₂, acids: H ₂SeO₃, H₂ SeO₄, Salts ―SeO₃, Nitride ―SeN₄, Selenium tetrahalides:
SeCL₄, SeF₄, SeBr₄, SeI₄ a. etc.

The reaction temperature is 20 to 200 degree C.

The intereaction time is 30 minutes to 24 hours.

The starting materials can be differences chemical products containing Selenium and liquid form of aliphatic halocarboxyl acids (hydrate or anhydrids ). Selenium compounds of the above-shown formulas can be obtained by conducting the above said reaction by using a hydrous solvent ( for Formula Nº1) with a water content solvent of preferably 0.05 to 15% or a anhydrous alcohol solvent about 1 to 20% (for Formula 2). The amount of hydrous or anhydrous solvents is not subject to any limitation and its ratio, they can be selected merely according to the technical or biologycal matter in carring out the reaction.

Through the reactions described above, the objective compounds can be obtained as an liquid product, suitable for production, analysis etc.

The final product of selenium complexes of haloethanoic acid hydrate and anhydrid and thier mixture is brown colored transparent, slightly viscose, liquid substances. The product has acidity approx. PH = 2,1 ― 2,5, LD₅₀ - approx. 2024 ― 2343 mg/kg. The concentration of selenium in product depends from type of starting chemical ingridients and can be between 0.001 ― 0,1%. Biologically the product is stable for many years when stored between 1 and 50_{°}C in closed brown glass containers. The medicine is practically nontoxic, its action is strongly localized, not absorbed into the organism, has no systemic, teratogenic, embryotoxic or carcinogenic properties.

The Selenium compounds of this invention are to be serve as pharmaceutical product for only topical use. As the form of medicaments , thay may take the form of solution in concetrations 1 to 100% (by weight), creams 3 to 40%, paste 3 to 40%, gele 3 to 40%, etc.

The recomended dosage per one lesion with diameter 0.1 to 2.0 cm a liquid form of this medicine is 0.01 to 0.2 cc. For multilesions the recomended dosage per one treatment can be 0.2 to 2.00 cc.

The clinical, histologycal, histochemical, cytologycal and statistical investigations of 366 volonter patients with differences types of skin lesions, treated with this invention, presented in Tabl. Nº 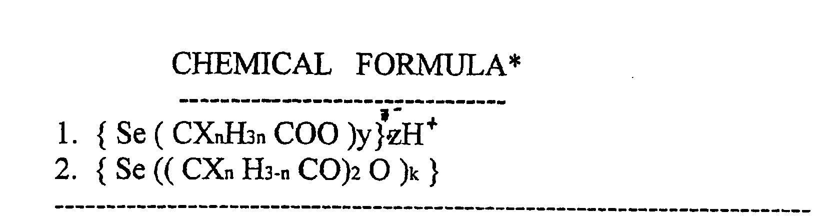
X = F; Cl; Br; I.
n = 0; 1; 2; 3.
y = 4 or 6.
z = 2 or 4.
k = 2 or 3.

## Claims

1. The novel Selenium complexes represented by general formulas (. No 1 and No 2).

2. A Selenium complexes according to claim 1 which is a Selenium complexes of haloethanoic acid hydrate,

3. A Selenium complexes according to claim 1 which is a Selenium complexes of haloethanoic acid anhydride.

4. The Selenium complexes as in claim 1, 2, 3, which are represented by mixed formulas 1 and 2.
